(19) Europäisches Patentamt
European Patent Office
Office européen des brevets

(11) Veröffentlichungsnummer: **0 005 192**
**B1**

(12) **EUROPÄISCHE PATENTSCHRIFT**

(45) Veröffentlichungstag der Patentschrift: **30.12.81**

(51) Int. Cl.³: **C 07 D 261/08,** A 61 K 31/41

(21) Anmeldenummer: **79101108.3**

(22) Anmeldetag: **11.04.79**

(54) **Alkylaminopropanolderivate von 3-Alkyl-5-(2-hydroxystyryl)-isoxazolen, Verfahren zu ihrer Herstellung und diese enthaltende therapeutische Mittel.**

(30) Priorität: **29.04.78 DE 2818999**

(43) Veröffentlichungstag der Anmeldung: **14.11.79 Patentblatt 79/23**

(45) Bekanntmachung des Hinweises auf die Patenterteilung: **30.12.81 Patentblatt 81/52**

(84) Benannte Vertragsstaaten: **BE CH DE FR GB IT LU NL SE**

(56) Entgegenhaltungen:
**CH-A-451 115**
**DE-A-2 007 751**
**US-A-3 624 079**

(73) Patentinhaber: **BASF Aktiengesellschaft, Carl-Bosch-Strasse 38, D-6700 Ludwigshafen (DE)**

(72) Erfinder: **Thieme, Peter C.,Dr., Dr.-Hans-Hoffmann-Strasse 5, D-6706 Wachenheim (DE)**
Erfinder: **Frickel, Fritz-Frieder, Dr., Prager Strasse 27, D-6700 Ludwigshafen (DE)**
Erfinder: **Theobald, Hans, Dr., Parkstrasse 2, D-6703 Limburgerhof (DE)**
Erfinder: **Franke, Albrecht, Dr., Mandelring 11, D-6706 Wachenheim (DE)**
Erfinder: **Lenke, Dieter, Dr., Kekuleplatz 1, D-6700 Ludwigshafen (DE)**
Erfinder: **Gries, Josef,Dr., Roemerweg 43, D-6706 Wachenheim (DE)**

**EP 0 005 192 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Alkylaminopropanolderivate von 3-Alkyl-5-(2-hydroxystyryl)-isoxazolen, Verfahren zu ihrer Herstellung und diese enthaltende therapeutische Mittel

Die vorliegende Erfindung betrifft neue Alkylaminopropanolderivate von 3-Alkyl-5-(2-hydroxystyryl)-isoxazolen und ihre physiologisch verträglichen Säureadditionssalze, Verfahren zu ihrer Herstellung und diese Verbindungen enthaltende pharmazeutische Zubereitungen, die als wertvolle Arzneimittel bei der Behandlung der Hypertonie, der coronaren Herzkrankheit und von Herzrhythmusstörungen verwendet werden können.

Es wurde gefunden, dass Verbindungen der allgemeinen Formel (I),

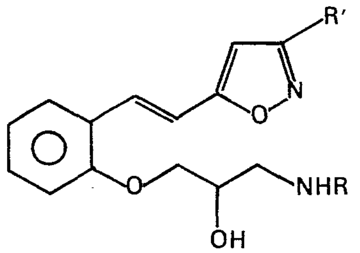

(I)

in der R Isopropyl, tert.-Butyl, einen Cyclopropylrest oder den 3-Methyl-1-butinyl-3-rest und R′ einen Alkylrest mit 1 bis 4 C-Atomen bedeuten, und ihre physiologisch verträglichen Säureadditionssalze wertvolle pharmakologische Eigenschaften aufweisen.

Als Alkylreste für R′ mit 1 bis 4 C-Atomen, geradkettig oder verzweigt, seien Methyl, Äthyl, Propyl, Isopropyl, n-Butyl, sek.-Butyl und tert.-Butyl genannt. Davon ist bevorzugt Methyl und Äthyl.

Dementsprechend können neben den in den Ausführungsbeispielen angegebenen Verbindungen als erfindungsgemässe Verbindungen beispielsweise genannt werden:

3-n-Butyl-5-[2-(2-hydroxy-3-isopropylamino-propoxy)-styryl]isoxazol

3-sec.-Butyl-5-[2-(2-hydroxy-3-isopropylamino-propoxy)-styryl]-isoxazol.

Die erfindungsgemässen Verbindungen können hergestellt werden, indem man ein 3-Alkyl-5-styryl-isoxazol der allgemeinen Formel II,

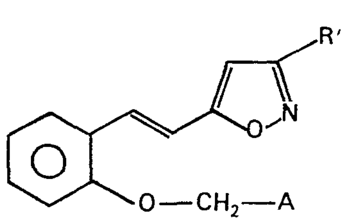

(II)

in der A den Rest

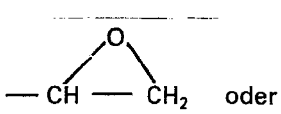

oder

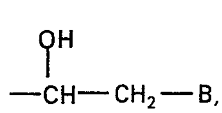

wobei B für eine nukleofuge Abgangsgruppe steht, bedeutet und R′ die für Formel I angegebenen Bedeutungen hat, mit einem Amin der allgemeinen Formel

$H_2N-R$,

in der R die für Formel I angegebenen Bedeutungen hat, zweckmässigerweise in einem Lösungsmittel und gegebenenfalls in Gegenwart eines säurebindenden Mittels in an sich bekannter Weise umsetzt und gegebenenfalls die erhaltene Verbindung in ein Säureadditionssalz einer physiologisch verträglichen Säure überführt.

Die Abgangsgruppe B stellt bevorzugt ein Halogenatom, insbesondere Chlor, Brom oder Jod, dar. Weiterhin kommen als nukleofuge Abgangsgruppen beispielsweise aromatische oder aliphatische Sulfonsäurereste in Betracht, wie der p-Toluolsulfonsäure-, der p-Brombenzolsulfonsäure- oder der Methansulfonsäurerest.

Die Umsetzungen werden bei Temperaturen von 10 bis 120°C, d.h. beim Raumtemperaturen oder bei höheren Temperaturen, zweckmässig bei Temperaturen von 50 bis 120°C, durchgeführt. Die Umsetzungen können unter Atmosphärendruck oder in einem geschlossenen Gefäss unter erhöhtem Druck gegebenenfalls unter Erwärmung auf den angegebenen Temperaturbereich durchgeführt werden.

Die Ausgangsverbindungen können direkt, d.h. ohne Zugabe eines Verdünnungs- oder Lösungsmittels, umgesetzt werden. Zweckmässigerweise werden die Umsetzungen jedoch in Gegenwart eines inerten Verdünnungs- oder Lösungsmittels, beispielsweise eines niederen Alkohols mit 1 bis 4 C-Atomen, wie Methanol, Äthanol oder Propanol, vorzugsweise Isopropanol oder Äthanol, eines niederen gesättigten Dialkyläthers, Dialkylglykoläthers oder cyclischen Äthers, wie Diäthyläther, 1,2-Dimethoxyäthan, Tetrahydrofuran oder Dioxan, eines Benzolkohlenwasserstoffs, wie Benzol selbst oder eines Alkylbenzols, insbesondere Toluol oder Xylol, oder eines aliphatischen Kohlenwasserstoffes, wie Hexan, Heptan oder Octan, eines niederen aliphatischen Ketons, wie Aceton, Methyläthylketon oder Methyl-isobutylketon, eines Dialkylformamids, wie Dimethyl- oder Diäthylformamid, von Dimethylsulfoxid oder in Gegenwart von Wasser oder in Mischungen der genannten Lösungsmittel durchgeführt.

Auch ist das in überschüssiger Menge verwendete Amin der Formel $H_2N-R$ gegebenenfalls als Verdünnungs- oder Lösungsmittel geeignet.

Bevorzugte Lösungsmittel bei der Umsetzung von 3-Alkyl-5-[2-(2,3-epoxy-propoxy)-styryl]-isoxazolen mit einem Amin $R-NH_2$ sind niedere Alkohole, insbesondere Äthanol oder Isopropanol, wobei die Umsetzung bevorzugt bei Temperaturen von 50°C bis 120°C durchgeführt wird. Gegebenenfalls kann die Umsetzung in einem geschlossenen Gefäss unter Druck durchgeführt

werden, insbesondere wenn niedrigsiedende Amine verwendet werden. Bei der nukleophilen Substitution eines Restes B sind als Lösungsmittel ein niederes aliphatisches Keton, wie Aceton, Methyläthylketon oder Methyl-isobutylketon, ein cyclischer aliphatischer Äther, insbesondere Tetrahydrofuran oder Dioxan, oder ein Dialkylformamid, wie Dimethylformamid, und Temperaturen von 90 bis 120°C bevorzugt. Gegebenenfalls wird die Reaktion in Gegenwart einer katalytischen Menge Natrium- oder Kaliumjodid durchgeführt.

Es sei erwähnt, dass als Ausgangsverbindung der Formel II gegebenenfalls auch eine Mischung des Epoxids mit einem Halogenhydrin in Betracht kommt, da bei der technischen Herstellung der Ausgangsverbindungen unter Umständen Gemische entstehen.

Bei einer zweckmässigen Ausführungsform zur nukleophilen Substitution des Restes B durch das verwendete Amin wird die Umsetzung in Gegenwart einer Base als säurebindendes Mittel durchgeführt. Bevorzuge Basen sind Alkalimetallhydroxide, -carbonate, -hydrogencarbonate, -alkoholate, insbesondere Methylate und Äthylate, oder ein tertiäres organisches Amin, wie Pyridin, oder ein Trialkylamin, wie Trimethylamin oder Triäthylamin. Von den Alkaliverbindungen kommen insbesondere die des Natriums und Kaliums in Betracht. Dabei wird die Base in stöchiometrischer Menge oder in geringem Überschuss verwendet. Gegebenenfalls ist es zweckmässig, das zur Umsetzung verwendete Amin $H_2N-R$ in überschüssiger Menge gleichzeitig als säurebindendes Mittel zu verwenden.

Die vollständige Umsetzung hängt von der Reaktionstemperatur ab und ist im allgemeinen innerhalb von 2 bis 15 Stunden beendet. Das Reaktionsprodukt kann in an sich üblicher Weise gewonnen werden, z.B. durch Filtration oder Abdestillieren des Verdünnungs- oder Lösungsmittels aus dem Reaktionsgemisch. Eine Reinigung der erhaltenen Verbindung erfolgt in üblicher Weise, beispielsweise durch Umkristallisieren aus einem Lösungsmittel, Überführen in eine Säureadditionsverbindung oder durch Säulenchromatographie.

Die Ausgangsverbindungen der Formel (II) können durch Alkylierung eines 3-Alkyl-5-(2-hydroxy-styryl)-isoxazols, die beispielsweise in einer Wittig-Reaktion aus einem Dialkyl-isoxazolyl-5-methylen-phosphonat und einem an der OH-Gruppe mit einer Schutzgruppe versehenen Salicylaldehyd gemäss der mit gleicher Priorität eingereichten europäischen Patentanmeldung Nr. 0 005 186 hergestellt werden, mit einem Epihalogenhydrin oder einem α,ω-Dihalogen-2-propanol erhalten werden. Als Epihalogenhydrine kommen Epichlorhydrin, Epibromhydrin und Epijodhydrin und als α,ω-Dihalogen-2-propanol kommen insbesondere 1,3-Dichlor-2-propanol und 1,3-Dibrom-2-propanol in Betracht.

Die Umsetzung der 3-Alkyl-5-(2-hydroxy-styryl)-isoxazole zur Herstellung der Ausgangsverbindungen der Formel III werden zweckmässigerweise bei Temperaturen von 0 bis 120°C und unter Normaldruck oder in einem geschlossenen Gefäss unter erhöhtem Druck durchgeführt. Die Umsetzungen werden zweckmässigerweise in einem inerten Verdünnungs- oder Lösungsmittel, z.B. einem niederen aliphatischen Keton, wie Aceton, Methyläthylketon oder Methyl-isobutylketon, einem niederen Alkohol mit 1 bis 4 C-Atomen, wie Methanol, Äthanol, Propanol oder Butanol, einem niederen aliphatischen oder cyclischen Äther, wie Diäthyläther, Tetrahydrofuran oder Dioxan, einem Dialkylformamid, wie Dimethylformamid oder Diäthylformamid, oder Dimethylsulfoxid und Hexamethylphosphortriamid oder mit überschüssigem Alkylierungsmittel als Verdünnungs- oder Lösungsmittel durchgeführt.

Bevorzugt werden die Umsetzungen in Gegenwart einer Base als säurebindendes Mittel vorgenommen. Geeignete Basen sind Alkalimetallcarbonate, -hydrogencarbonate, -hydroxide, -hydride oder -alkohalate, insbesondere des Natriums und Kaliums, basische Oxide, wie Aluminiumoxid oder Calciumoxid, organische tertiäre Basen, wie Pyridin, Piperidin oder niedere Trialkylamine, wie Trimethyl- der Triäthylamin. Dabei können die Basen im Verhältnis zum eingesetzten Alkylierungsmittel in katalytischer Menge oder in stöchiometrischer Menge bzw. in geringem Überschuss verwendet werden.

Bevorzugt werden die 3-Alkyl-5-(2-hydroxystyryl)-isoxazole mit Epibromhydrin oder 1,2-Dibrompropanol-2 in einem polaren aprotischen Lösungsmittel, insbesondere Dimethylsulfoxid, in Gegenwart von mindestens einem Moläquivalent Base, insbesondere Natriumhydrid, bezogen auf das Alkylierungsmittel, bei Temperaturen von 0 bis 50°C umgesetzt.

Analog des in Liebigs Annalen der Chemie 1976, Seiten 221 bis 223, beschriebenen Verfahrens für die Umsetzung von Phenol mit 1,3-Dichlor-2-propanol können auch 3-Alkyl-5-(2-hydroxy-styryl)-isoxazole mit der äquivalenten Menge 1,3-Dichlor-2-propanol in wässriger Natronlauge bei Temperaturen um 50°C umgesetzt werden.

Die Ausgangsverbindungen der allgemeinen Formel II, in denen A den Rest

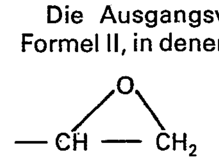

bedeutet, können auch erhalten werden, indem man einen Methanphosphonsäureester der allgemeinen Formel VI

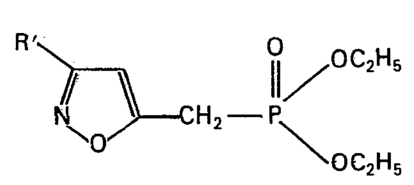

(VI)

mit dem in J. Chem. Soc. London 1974, 1571-1577 beschriebenen o-(2,3-Epoxypropoxy)-benzaldehyd umsetzt.

Zur Reaktion von o-(2,3-Epoxypropoxy)-benzaldehyd mit einem Phosphoran der Formel VI kommen als Reaktionsmedium inerte organische Lösungsmittel in Betracht, wie z.B. ein niederer gesättigter Dialkyläther, Dialkylglykoläther oder cyclischer Äther, wie Diäthyläther, 1,2-Dimethyloxyäthan, Tetrahydrofuran oder Dioxan, Benzol oder ein Alkylbenzol, wie Toluol oder Xylol, ein aliphatischer Kohlenwasserstoff, wie Hexan, Heptan oder Octan, Dimethylsulfoxid, Dimethylformamid oder Mischungen der genannten Lösungsmittel. Die Umsetzungen werden zwischen 0°C und den Siedetemperaturen der verwendeten Lösungsmittel, zweckmässigerweise bei Raumtemperatur, innerhalb von 1 bis 48 Stunden, vorzugsweise 1 bis 16 Stunden und vorteilhaft in einer Stickstoffatmosphäre durchgeführt. Geeignete Basen für diese Wittig-Horner-Reaktion sind Alkalimetallhydride, -amide oder -alkoholate, insbesondere die des Natriums und Kaliums, vorzugsweise verwendet man Natriummethylat.

Die erfindungsgemässen Verbindungen der Formel (I) besitzen am Kohlenstoffatom 2 der aliphatischen Seitenkette ein Chiralitätszentrum und werden als Racemate erhalten, die durch bekannte Methoden, beispielsweise durch Bildung diastereomerer Salze mit optisch aktiven Hilfssäuren, wie Dibenzoylweinsäure, Campher-10-sulfonsäure, Ditoluylweinsäure oder 3-Bromcampher-8-sulfonsäure, in die optisch aktiven Antipoden getrennt werden können.

Gegebenenfalls werden die erhaltenen erfindungsgemässen Verbindungen in das Säureadditionssalz einer physiologisch verträglichen Säure überführt. Als übliche physiologisch verträgliche organische oder anorganische Säuren kommen beispielsweise Salzsäure, Bromwasserstoffsäure, Phosphorsäure oder Schwefelsäure und als organische Säuren beispielsweise Oxalsäure, Maleinsäure, Fumarsäure, Milchsäure, Weinsäure, Äpfelsäure, Zitronensäure, Salicylsäure, Adipinsäure oder Benzoesäure in Betracht. Weitere geeignete Säuren können aus Fortschritte der Arzneimittelforschung Band 10, Seiten 223 bis 225, Birkhäuser Verlag, Basel und Stuttgart, 1966, oder aus Journal of Pharmaceutical Sciences, Volume 66, Seiten 1 bis 5 (1977), entnommen werden.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Äthanol oder Propanol oder einem niederen Keton wie Aceton, Methyläthylketon oder Methylisobutylketon oder einem Äther wie Diäthyläther, Tetrahydrofuran oder Dioxan, erhalten. Zur besseren Kristallabscheidung können auch Mischungen der genannten Lösungsmittel verwendet werden. Darüber hinaus können pharmazeutisch vertretbare wässrige Lösungen von Säure-Additionsverbindungen der Aminpropanol-Derivate der allgemeinen Formel (I) durch Auflösen der freien Base der allgemeinen Formel (I) in einer wässrigen Säurelösung hergestellt werden.

Die erfindungsgemässen Verbindungen und ihre physiologisch verträglichen Säureadditionssalze weisen wertvolle pharmakologische Eigenschaften auf. Sie sind pharmakodynamisch als hochaktive β-Sympatholytika mit akuter blutdrucksenkender bzw. antihypertensiver Wirkung zu charakterisieren. Dieser Wirkungstyp ist insofern ungewöhnlich, als bekannte β-Sympatholytika, z.B. Propranolol, nur bei länger dauernder Verabreichung antihypertensiv wirken.

Aufgrund der genannten Effekte sind die Verbindungen pharmakotherapeutisch besonders zur Behandlung der Hypertonie, der coronaren Herzkrankheit und von Herzrhythmusstörungen geeignet.

Die Untersuchung der pharmakodynamischen Eigenschaften wurde nach folgenden Methoden vorgenommen:

1. β-sympatholytische Wirkung an der Isoproterenol-Tachycardie der narkotisierten Katze.

i.v. Applikation von 0,001 mg/kg des β-Sympathomimetikums Isoproterenol steigert die Herzfrequenz von Bastardkatzen (Gewicht: 2-4 kg) in Hexobarbital-Narkose (200 mg/kg i.m.) um durchschnittlich 61 Schläge (40%).

β-Sympatholytika hemmen diese Frequenzsteigerung spezifisch und dosisabhängig. Die Applikation der getesteten Substanzen erfolgt 10 min vor Isoproterenol an Gruppen von 3 bis 5 Katzen pro Dosis.

Als ED 50% wird die Dosis ermittelt, welche die Isoproterenol-Tachycardie um 50% hemmt.

2. Blutdrucksenkende Wirkung an der narkotisierten Ratte. Zur Testung der blutdrucksenkenden Wirkung erhalten Gruppen von 3 bis 5 männlichen Sprague-Dawley-Ratten (Gewicht: 230–280 g) in Urethan-Narkose (1,78 g/kg i.p.) die Substanzen intravenös appliziert.

Die Messung des Blutdrucks in der A. carotis erfolgt über Statham-Transducer.

Als ED 20% wird die Dosis ermittelt, welche den mittleren Carotisdruck um 20% senkt.

3. Antihypertensive Wirkung an spontan hypertonen Ratten. Die Substanzen werden Gruppen von 8 männlichen spontan hypertonen Okamoto-Ratten (Gewicht: 280–350 g) oral appliziert. Vor und zwei Stunden nach der Applikation wird der systolische Blutdruck am Rattenschwanz unblutig mit Hilfe von Piezokristallaufnehmern gemessen.

Als ED 20% wird unter Berücksichtigung der Werte unbehandelter Kontrolltiere die Dosis bestimmt, welche den systolischen Druck um 20% senkt.

4. Akute Toxizität an der Maus. Zur Bestimmung der akuten Toxizität (LD 50) werden die Substanzen Gruppen von je 10 weiblichen NMRI-Mäusen (Gewicht: 19–20 g) intraperitoneal appliziert. Die Beobachtungsdauer beträgt 1 Woche.

Die Berechnung der wirksamen Dosen (s. 1.–3.) erfolgte aus den linearen Beziehungen zwischen den Logarithmen der Dosen und der Wirkung mit Hilfe der Regressionsanalyse. Die LD 50 (4.) wurden mit Hilfe der Probit-Analyse bestimmt. Als Referenzsubstanz diente das bekannte β-Sympatholytikum Propanolol.

Wie aus der Tabelle 1 hervorgeht, ist die β-sympatholytische Wirksamkeit der Beispiele 1, 3, 6 und 13 6,1- bis 20mal höher als die des bekannten β-Sympatholytikums Propanolol. Beispiel 5 ist dem Propanolol etwa wirkungsgleich.

Zusätzlich zu diesem Effekt tritt bei den erfindungsgemässen Substanzen eine akute Senkung des arteriellen Blutdrucks auf. Nach Applikation von 0,4 mg/kg Beispiel 3 wird an der Ratte eine Drucksenkung um durchschnittlich 20% beobachtet. In gleicher Weise senken die Beispiele 1, 5, 6 und 13 den Blutdruck in Dosen zwischen 0,65 und 1 mg/kg (ED 20%). Propanolol wirkt im Gegensatz dazu an Ratten bis zu Dosen von 2,15 mg/kg blutdrucksteigernd. Erst die subletale Dosis 4,64 mg/kg senkt den Druck um durchschnittlich 36%. Die etwa doppelte Dosis (10 mg/kg) tötet 2 von 6 Tieren. Nach Applikation von 10 mg/kg Beispiel 3 stirbt 1 von 6 Tieren. Diese Dosis ist jedoch 25 mal höher als die blutdrucksenkende Dosis (0,4 mg/kg).

Wie für Beispiel 3 nachgewiesen wurde, wirken die Substanzen auch an spontan hypertonen Ratten nach oraler Applikation blutdrucksenkend. 14,5 mg/kg senken den erhöhten Blutdruck um durchschnittlich 20%. Propanolol ist in diesem Test bis zu einer Dosis von 100 mg/kg wirkungslos.

Die letale Dosis (LD 50, Maus, i.p.) von Beispiel 3 (123 mg/kg) liegt etwas höher, die von Beispiel 13 mehr als doppelt so hoch wie die des Propanolol (108 mg/kg). Die Beispiele 1, 5 und 6 sind ebenso toxisch oder etwas toxischer als Propanolol. In Anbetracht der hohen Wirksamkeit (Beispiel 1 und 6) bzw. des insgesamt neuartigen Wirkungstyps ist dieser Befund ohne Relevanz.

Die Tabelle 1 enthält auch einen Vergleich zu der Verbindung des Beispiels 4 der DE-A-2 007 751: 3-Isopropylamino-1-p-(β-methoxycarbonylvinyl) phenoxy-2-propanolol. Die Werte zeigen, dass die Vergleichssubstanz aus DE-A-2 007 761 wesentlich weniger wirksam als das anerkannte Mittel Propanolol ist.

Tabelle 1

| Beispiel Nr. | β-Sympatholytische Wirkung 1) | | Blutdrucksenkende Wirkung 2) | Letalität 4) |
|---|---|---|---|---|
| | ED 50% | R.W. | ED 20% | LD 50 |
| 1 | 0,0077 | 14,3 | 0,65 | 108 |
| 3 | 0,0055 | 20,0 | 0,40 | 123 |
| 5 | 0,12 | 0,9 | 0,83 | 95 |
| 6 | 0,018 | 6,1 | 0,93 | 84 |
| 13 | 0,017 | 6,5 | 1,00 | 247 |
| Propanolol | 0,11 | 1,0 | 3) | 108 |
| DE-A-2007751 Beispiel 4 | 2,16 | 0,05 | >1,0 | – |

1) Katze. Hexobarbital-Narkose. Appl.: i.v. ED 50% = Dosis mg/kg, welche die Steigerung der Herzfrequenz durch Isoproterenol um 50% hemmt. R.W. = Relative Wirksamkeit. Propanolol = 1,00.

2) Ratte. Urethan-Narkose. Appl.: i.V. ED 20% = Dosis mg/kg, welche den Blutdruck um 20% senkt.

3) Bis 2,15 mg/kg Blutdrucksteigerung (11%); 4,64 mg/kg Blutdrucksenkung (36%); 10 mg/kg 2/6 Tieren gestorben.

4) Maus. Appl.: i.p. LD 50 mg/kg.

Gegenstand der vorliegenden Erfindung sind demnach auch therapeutische Mittel oder Zubereitungen, die neben üblichen Träger- und Verdünnungsmitteln eine Verbindung der Formel I als Wirkstoff enthalten, sowie die Verwendung der neuen Verbindungen zu therapeutischen Zwecken.

Die therapeutischen Mittel bzw. Zubereitungen werden mit den üblichen Trägerstoffen oder Verdünnungsmitteln und den üblicherweise verwendeten pharmazeutisch-technischen Hilfsstoffen entsprechend der gewünschten Applikationsart mit einer geeigneten Dosierung in bekannter Weise hergestellt.

Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen, wie Injektionslösungen, in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Mangesiumstearat oder Talk und/oder Mitteln zur Erzielung des Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit den erfindungsgemässen Wirkstoffen können zusätzlich geschmackverbessernde Mittel wie Saccharin, Cyclamat oder Zucker sowie z.B. Aromastoffe wie Vanillin oder Orangenextrakt, enthalten. Sie können ausserdem Suspendierhilfsstoffe wie Natriumcarboxymethylcellulose oder Konservierungsstoffe wie p-Hydroxy-benzoate, enthalten. Wirkstoffe enthaltende Kapseln können beispielsweise hergestellt werden, indem man den Wirkstoff mit einem inerten Träger wie Milchzukker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten, herstellen.

Als Einzeldosis der erfindungsgemässen Verbindungen kommen 1 bis 100 mg, vorzugsweise 4 bis 50 mg, an Menschen in Betracht.

Bezüglich ihrer Wirksamkeit sind besonders hervorzuheben:

3-Methyl-5-[2-(2-hydroxy-3-isopropylamino-propoxy)-styryl]-isoxazol,
3-Methyl-5-[2-(2-hydroxy-3-tert.-butylamino-propoxy)-styryl]-isoxazol,
3-Äthyl-5-[2-(2-hydroxy-3-tert.-butylamino-propoxy)-styryl]-isoxazol,
3-Äthyl-5-[2(2-hydroxy-3-cyclopropylamino-propoxy)-styryl]-isoxazol,
3-Methyl-5-[2-(2-hydroxy-3-(3-methyl-1-butin-3-ylamino)-propoxy-styryl]-isoxazol.

Die vorliegende Erfindung wird durch die nachfolgenden Ausführungsbeispiele näher erläutert.

Herstellung von Vorprodukten

Verbindung I: o-(α-Methoxy-äthoxy)-benzaldehyd

a) 610 g (5 Mol) Salicylaldehyd werden in 1,5 l Xylol gelöst; zu dieser Lösung werden bei 40–50°C 900 g (5 Mol) einer 30%igen $NaOCH_3$-Lösung in Methanol zugetropft. Anschliessend wird aufgeheizt und das Methanol abdestilliert, welches im Reaktionskolben nach und nach durch die gleiche Menge Xylol ersetzt wird. Es wird so lange erhitzt, bis das Xylol abzudestillieren beginnt (ca. 130°C am Destillationsübergang). Dann wird die Suspension des Na-Salzes des Salicylaldehyds auf 60°C abgekühlt und wie unter c) beschrieben weiter umgesetzt.

b) 200 ml Xylol werden mit einer Spatelspitze Hydrochinon versetzt und auf −20 bis −30°C abgekühlt und 290 g (5 Mol) Vinyl-methyläther einkondensiert. Anschliessend werden bei −30°C 183 g (5 Mol) HCl eingegast und die Lösung durch Stehenlassen auf Raumtemperatur erwärmt. Die Lösung des erhaltenen 1-Chloräthyl-methyläthers wird wie unter c) beschrieben weiter umgesetzt.

c) Die wie unter b) beschrieben hergestellte Lösung des 1-Chloräthylmethyläthers wird zu der 60°C heissen Lösung des Na-Salzes des Salicylaldehyds (siehe a) zugetropft und noch ca. 1½ Stunden bei 60°C weitergerührt, gegebenenfalls wird mit Hilfe von 30%iger $NaOCH_3$-Lösung ein pH-Wert von 8–9 eingestellt und über Nacht bei Raumtemperatur weitergerührt.

Anschliessend wird vom ausgefallenen Natriumchlorid abfiltriert, nachgewaschen mit Xylol und am Rotationsverdampfer das Xylol abdestilliert. Der verbleibende Rückstand wird bei 2 mm Hg über eine Kolonne destilliert. Es werden 690 g o-(α-Methoxy-äthoxy)-benzaldehyd vom $Kp_2$: 94–96°C gewonnen.

Verbindung II: (3-Methylisoxazolyl-5)-methanphosphonsäure-diäthylester

445 g 5-Chlormethyl-3-methylisoxazol und 674 g Phosphorigsäuretriäthylester werden langsam auf 150°C erhitzt und 4 Stunden bei dieser Temperatur belassen. Nach der Destillation erhält man 546 g (69% d. Theorie) (3-Methyl-isoxazolyl-5)-methanphosphon-säurediäthylester vom Sdp. 118–121°C/0,3 Torr.

$^1H$-NMR-Spektrum ($CHCl_3$, TMS intern):

τ = 3,85 (d, J = 3 Hz, 1H), 4,17 (m, J = 8 Hz, 4H), 6,67 (d, J = 22 Hz, 2H), 7,72 (s, 3H), 8,67 (t, J = 8 Hz, 6H).

$C_9H_{16}NO_4P$ (233,21)

| | | | | |
|---|---|---|---|---|
| Ber.: | C 46,35% | H 6,91% | N 6,01% | P 13,28% |
| Gef.: | C 45,9% | H 7,0% | N 6,0% | P 13,0% |

Verbindung III: (3-Äthylisoxazolyl-5)-methanphosphonsäure-diäthylester

15 g 5-Chlormethyl-3-äthyl-isoxazol und 18 g Triäthylphosphit werden langsam auf 150°C aufgeheizt und 2½ Stunden bei dieser Temperatur belassen. Nach dem Abkühlen wird der Rückstand unter vermindertem Druck destilliert. Man erhält 18,2 g (3-Äthylisoxazolyl-5)-methan-phosphonsäurediäthylester vom Sdp. 120–121°C/0,2 mm Hg (Ausbeute: 71,2%).

$^1H$-NMR-Spektrum ($CDCl_3$, TMS intern):

τ = 3,85 (d, J = 3 Hz, 1H), 4,17 (m, J = Hz, 4H), 6,60 (d, H = 20 Hz, 2H), 7,35 (q, J = Hz, 2H), 8,50–8,93 (m, 9H).

$C_{10}H_{18}NO_4P$ (247,23)

| | | | | |
|---|---|---|---|---|
| Ber.: | C 48,58% | H 7,34% | N 5,67% | P 12,53% |
| Gef.: | C 48,4% | H 7,1% | N 5,7 % | P 12,3 %. |

Die weiteren Phosphonester werden analog hergestellt.

(3-Isopropyl-isoxazolyl-5)-methanphosphonsäurediäthylester

$KP_{0,3}$: 117–122°C Ausbeute 73%

(3-tert.-Butyl-isoxazolyl-5)-methanphosphonsäurediäthylester

$Kp_{0,3}$: 126–132°C Ausbeute 88%.

Herstellung von Ausgangsverbindungen

Beispiel I

3-Methyl-5-(2-hydroxy-styryl)-isoxazol

8,8 g (0,2 Mol) 55% Natriumhydridsuspension in Paraffinöl werden in 100 ml absolutem Dimethylsulfoxid vorgelegt. Dazu werden 47 g (0,2 Mol) Diäthyl-(3-methyl-isoxazolyl-5)-methylenphosphonat bei Raumtemperatur getropft. Man lässt 30 min nachrühren und tropft anschliessend unter Rühren 36 g (0,2 Mol) o-(1-Methoxy-äthoxy)-benzaldehyd zu und lässt das Reaktionsgemisch für 24 Std. bei Raumtemperatur rühren. Man giesst auf 1 l Eiswasser und extrahiert 3 mal mit je 80 ml Methylenchlorid. Die vereinigten organischen Phasen werden mit Natriumsulfat getrocknet und am Rotationsverdampfer eingeengt. Der ölige Rückstand wird in 80 ml Methanol und 10 ml Wasser gelöst und mit 2 ml 5 n HCl versetzt und für 10 min nachgerührt. Dann wird langsam ein Überschuss an Wasser zum Gemisch gegeben, bis ein Niederschlag ausfällt. Dieser wird abgesaugt, mit Wasser gewaschen und aus Äthanol umkristallisiert. 19 g (47% d. Th.) farblose Kristalle, Fp. 236–238°C.

$C_{12}H_{11}NO_1$ (201)

ber. C 71,6 H 5,5 N 7,0
gef. C 71,8 H 5,5 N 6,8

Beispiel II

3-Äthyl-5-(2-hydroxy-styryl)-isoxazol

Gemäss I werden 6 g (0,02 Mol) Diäthyl-(3-äthyl-isoxazolyl-5)-methylenphosphonat und 4,4 g (0,02 Mol) o-(1-Methoxy-äthoxy)-benzaldehyd umgesetzt und aus Isopropanol umkristalliert. 1,7 g (32% d. Th.) farblose Kristalle.

Fp. 175–176°C.

$C_{13}H_{13}NO_2$ (215)

ber. C 72,5 H 6,1 N 6,5
gef. C 72,5 H 6,2 N 6,6

Beispiel III

3-Isopropyl-5-(2-hydroxy-styryl)-isoxazol

Gemäss I werden 32 g (0,12 Mol) Diäthyl-(3-isopropyl-isoxazolyl-5)-methylen-phosphonat und 22 g (0,12 Mol) o-(1-Methoxy-äthoxy)-benzaldehyd umgesetzt und aus Toluol umkristallisiert. 20 g (73% d. Th.) farblose Kristalle.

Fp. 129–133°C.

$C_{16}H_{15}NO_2$ (229)

ber. C 73,3 H 6,6 N 6,1
gef. C 73,7 H 6,7 N 5,8

Beispiel IV

3-tert.-Butyl-5-(2-hydroxy-styryl)-isoxazol

Gemäss I werden 35 g (0,13 Mol) Diäthyl-(3-tert.-butyl-isoxazolyl-5)-methylen-phosphonat und 23 g (0,13 Mol) o-(1-Methoxy-äthoxy)-benzaldehyd umgesetzt und am Toluol umkristallisiert. 24,8 g (78% d. Th.) farblose Kristalle.

Fp. 152–155°C.

$C_{15}H_{17}NO_2$ (243)

ber. C 74,0 H 7,0 N 5,8
gef. C 73,4 H 7,3 N 5,5

Beispiel V

3-Methyl-5-[2-(2-(2,3-epoxypropoxy)-styryl]-iosoxazol

6,44 g 55%iges Natriumhydrid in Paraffinöl (0,15 Mol) werden in 200 ml absolut Dimethylsulfoxid vorgelegt und tropfenweise bei Raumtemperatur mit 30 g (0,15 Mol) 3-Methyl-5-(2-hydroxy-styryl)-isoxazol, in 50 ml Dimethylsulfoxid gelöst, versetzt. Nach beendeter Wasserstoffentwicklung werden 20,2 g (0,15 Mol) Epibromhydrin zugetropft und das Reaktionsgemisch für 20 Stunden bei Raumtemperatur gerührt. Man gibt das Gemisch auf 1,5 l Eiswasser, der feste Rückstand wird abgesaugt und aus Isopropanol umkristallisiert. 26,2 g (68% d. Th.) farblose Kristalle, Fp. 99–100°C.

$C_{15}H_{15}NO_3$ (257)

ber.: C 70,0 H 5,9 N 5,4
gef.: C 70,0 H 5,9 N 5,5

Beispiel VI

3-Äthyl-5-[2-(2,3-epoxypropoxy)-styryl]-isoxazol

Wird analog V aus 5,1 g 55%igem Natriumhydrid (0,116 Mol), 25,0 g (0,116 Mol) 3-Äthyl-5-(2-hydroxy-styryl)-isoxazol und 15,9 g (0,116 Mol) Epibromhydrin hergestellt. Das Reaktionsgemisch wird auf Kochsalzlösung gegossen und mit Diäthyläther ausgeschüttelt. Die ätherische Lösung wird mit wasserfreiem Natriumsulfat getrocknet und eingeengt. Man erhält 29,2 g (93% d. Th.) farbloses Öl.

Beispiel VII

3-Isopropyl-5-[2-(2,3-epoxypropoxy)-styryl]-isoxazol

Wird analog VI aus 3,4 g 55%igem Natriumhydrid (0,078 Mol), 18 g (0,078 Mol) 3-Isopropyl-5-(2-hydroxystyryl)-isoxazol und 10,8 g (0,078 Mol) Epibromhydrin hergestellt. 21,5 g (97% d. Th.) farbloses Öl.

Beispiel VIII

3-tert.-Butyl-5-[2-(2,3-epoxypropoxy)-styryl]-isoxazol

Wird analog VI aus 3,8 g 55%igem Natriumhydrid (0,086 Mol), 21 g (0,086 Mol) 3-tert.-Butyl-5-(2-hydroxystyryl)-isoxazol und 11,8 g (0,086 Mol)

Epibromhydrin hergestellt. 25,0 g (97% d. Th.) farbloses Öl.

Beispiel IX
1,0 g 3-Methyl-5-[2-(2,3-epoxypropoxy)-styryl]-isoxazol wird in 20 ml 3-N-ätherischer Chlorwasserstofflösung gelöst und 12 Stunden bei Raumtemperatur belassen. Der gebildete, harzartige Anteil wird abgetrennt und mit Chloroform über Kieselgel chromatographiert. Die Produkteluate werden im Vakuum zur Trockene eingeengt und anschliessend wird der Rohaustrag aus einem Aceton-Cyclohexan-Gemisch umkristallisiert. Man erhält NMR-spektroskopisches reines (3-Methyl-5-[2-(2-hydroxy-3-chlorpropoxy)-styryl]-isoxazol) vom Schmelzpunkt 67–68°C.

$^1H$-NMR-Spektrum ($CDCl_3$, TMS intern).

$\tau$ = 2,30 3,15 (m, 6H), 3,92 (s, 1H), 5,60–5,92 (m, 3H), 6,24 (d, J = 3,5 Hz, 2H), 6,77 (breites s, OH)

Herstellung von erfindungsgemässen Verbindungen:

Beispiel 1
3-Methyl-5-[2-(2-hydroxy-3-isopropyl-aminopropoxy)-styryl]-isoxazol:
3,4 g (0,013 Mol) 3-Methyl-5-[2-(2,3-epoxy-propoxy)-styryl]-isoxazol und 2,3 g (0,039 Mol) Isopropylamin werden in 50 ml Isopropanol für 8 Std. zum Rückfluss erhitzt. Nach dem Abkühlen wird die Reaktionslösung filtriert und am Rotationsverdampfer eingeengt. Man erhält 3,8 g festen Rückstand, der aus Toluol umkristallisiert wird. 2,4 g (58% d. Th.) farblose Kristalle, Fp. 106–108°C.

$C_{18}H_{24}N_2O_3$ (316,4)

ber.: C 68,3 H 7,6 N 8,9
gef.: C 68,4 H 7,6 N 9,0

Beispiel 2
3-Methyl-5-[2-(2-hydroxy-3-cyclopropylamino-propoxy)-styryl]-isoxazol:

Wird analog Beispiel 1 aus 5,0 g (0,019 Mol) 3-Methyl-5-[2-(2,3-epoxypropoxy)-styryl]-isoxazol und 1,21 g (0,2 Mol) Cyclopropylamin erhalten. Aus Toluol 2,4 g (40% d. Th.) farblose Kristalle. Fp. 108–109°C.

$C_{18}H_{22}N_2O_3$ (314,4)

ber.: C 68,8 H 7,1 N 8,9
gef.: C 68,8 H 7,0 N 8,9

Beispiel 3
3-Methyl-5-[2-(2-hydroxy-3-tert.-butylaminopropoxy)-styryl]-isoxazol:

Wird analog Beispiel 1 aus 5,0 g (0,019 Mol) 3-Methyl-5-[2-(2,3-epoxypropoxy)-styryl]-isoxazol und 1,56 g (0,2 Mol) tert.-Butylamin erhalten. 3,1 g (48% d. Th.) farblose Kristalle, Fp. 83–85°C.

$C_{19}H_{27}N_2O_3 \cdot 0,5\ H_2O$ (339,4)

ber.: C 67,3 H 8,0 N 8,3
gef.: C 67,2 H 7,9 N 8,1

30 g rohes 3-Methyl-5-[2-(2-hydroxy-3-tert.-butylamino-propoxy)-styryl]-isoxazol werden in wenig Äthanol gelöst und tropfenweise bis zur vollständigen Ausfällung mit ätherischer Chlorwasserstofflösung versetzt. Das ausgefallene 3-Methyl-5-[2-(2-hydroxy-3-tert.-butylamino-propoxy)-styryl]-isoxazol-hydrochlorid wird abgesaugt, mit trockenem Äther gewaschen, zweimal aus Methanol-Äther-Gemisch (Vol. 1:1) umkristallisiert und getrocknet.
Ausbeute: 33,8 g (79% d. Theorie) vom Schmelzpunkt 217°C.

$C_{19}H_{27}O_3N_2Cl$ (367)

ber.: C 62,2 H 7,4 N 7,6 Cl 9,7
gef.: C 62,1 H 7,3 N 7,7 Cl 9,8

In gleicher Weise wird mit ätherischer Fumarsäure das neutrale Fumarat erhalten: Schmelzpunkt 188°C.

Beispiel 4
3-Äthyl-5-[2-(2-hydroxy-3-isopropylaminopropoxy)-styryl]-isoxazol:

Wird aus 7 g (0,026 Mol) 3-Äthyl-5-[2-(2,3-epoxypropoxy)-styryl]-isoxazol und 2,4 g Isopropylamin (0,04 Mol) in 100ml Isopropanol hergestellt. Man erhält 8,1 g Öl, das in 100 ml Isopropanol gelöst und mit ätherischer Salzsäure versetzt wird. Die ausgefallenen Kristalle werden abgesaugt und getrocknet.
4,9 g (50% d. Th.) Fp. 134–135°C.

$C_{19}H_{25}ClN_2O_3$ (366,9)

ber.: C 62,7 H 7,4 N 7,6 Cl 9,7
gef.: C 61,9 H 6,9 N 7,7 Cl 9,6

Beispiel 5
3-Äthyl-5-[2-(2-hydroxy-3-cyclopropylaminopropoxy)-styryl]-isoxazol:

7 g (0,026 Mol) 3-Äthyl-5-[2-(2,3-epoxypropoxy)-styryl]-isoxazol und 2,3 g Cyclopropylamin (0,04 Mol) werden in 100 ml Isopropanol analog Beispiel 1 umgesetzt. Man erhält 8,6 g eines Öls, das an einer Säule (55×5 cm) von Kieselgel 60 (0,063–0,20 mm) der Fa. Merck von Verunreinigungen getrennt. Das Elutionsmittel ist ein 9:1-Gemisch aus Methylenchlorid und Methanol. Die Fraktionen werden dünnschichtchromatographisch auf Reinheit geprüft. Man erhält 3,2 g Öl, das in 40 ml Isopropanol und wenig Äther gelöst wird und mit 1,1 g Fumarsäure, gelöst in heissem Isopropanol gefällt wird. Die Kristalle werden abgesaugt und getrocknet.

2,1 g (21% d. Th.), Fp. 137–139°C.

$C_{22}H_{26}N_2O_5$ (386,5)

ber.: C 65,2 H 6,7 N 7,2
gef.: C 64,7 H 6,8 N 7,5

Beispiel 6
3-Äthyl-5-[2-(2-hydroxy-3-tert.-butylaminopropoxy)-styryl]-isoxazol:

7 g (0,026 Mol) 3-Äthyl-5-[2-(2,3-epoxypropoxy)-styryl]-isoxazol und 3,0 g (0,04 Mol) tert.-Butylamin werden analog Beispiel 4 umgesetzt. 6,6 g (66% d. Th.), Fp. 163–164°C.

$C_{20}H_{29}ClN_2O_3$

ber.: C 63,1 H 7,7 N 7,3 Cl 9,3
gef.: C 62,9 H 7,5 N 7,2 Cl 9,2

Beispiel 7
3-Isopropyl-5-[2-(2-hydroxy-3-isopropylamino-propoxy)-styryl]-isoxazol:

7 g (0,025 Mol) 3-Isopropyl-5-[2-(2,3-epoxypropoxy)-styryl]-isoxazol und 3 g (0,05 Mol) Isopropylamin werden analog Beispiel 1 umgesetzt.
Aus Toluol erhält man 5,9 g (68% d. Th.) farblose Kristalle. Fp. 87–88°C.

$C_{20}H_{28}N_2O_3$ (344,5)

ber.: C 69,7 H 8,2 N 8,1
gef.: C 69,9 H 8,2 N 8,1

Beispiel 8
3-Isopropyl-5-[2-(2-hydroxy-3-cyclopropylamino propoxy)-styryl]-isoxazol:

7 g (0,025 Mol) 3-Isopropyl-5-[2-(2,3-epoxypropoxy)-styryl]-isoxazol und 3 g (0,05 Mol) Cyclopropylamin werden analog Beispiel 5 umgesetzt. Man erhält 3,0 g (30% d. Th.) farblose Kristalle als Fumarat. Fp. 148–152°C.

$C_{22}H_{28}N_2O_5$ (400,5)

ber.: C 66,0 H 7,0 N 7,0
gef.: C 65,7 H 6,8 N 7,0

Beispiel 9
3-Isopropyl-5-[2-(2-hydroxy-3-tert.-butylamino-propoxy)-styryl]-isoxazol:

7 g (0,025 Mol) 3-Isopropyl-5-[2-(2,3-epoxy-propoxy)-styryl]-isoxazol und 3,6 g (0,05 Mol) tert.-Butylamin werden analog Beispiel 4 umgesetzt. Man erhält 5,8 g (59% d. Th.) farblose Kristalle. Fp. 191–193°C.

$C_{21}H_{31}ClN_2O_3$ (394,9)

ber.: C 63,9 H 7,9 N 7,1 Cl 9,0
gef.: C 64,1 H 8,0 N 6,9 Cl 9,1

Beispiel 10
3-tert.-Butyl-5-[2-(2-hydroxy-3-isopropylamino-propoxy)-styryl]-isoxazol:

7 g (0,023 Mol) 3-tert.-Butyl-5-[2-(2,3-epoxy-propoxy)-styryl]-isoxazol und 2,8 g (0,05) Isopropylamin werden analog Beispiel 5 umgesetzt. Man erhält 2,5 g (27% d. Th.) farblose Kristalle als Fumarat. Fp. 192–194°C.

$C_{25}H_{34}N_2O_5$ (474,6)

ber.: C 66,3 H 7,6 N 6,7
gef.: C 66,5 H 7,8 N 6,6

Beispiel 11
3-tert.-Butyl-5-[2-(2-hydroxy-3-cyclopropylaminopropoxy)-styryl]-isoxazol:

7 g (0,023 Mol) 3-tert.-Butyl-5-[2-(2,3-epoxy-propoxy)-styryl]-isoxazol und 2,7 g (0,047 Mol) Cyclopropylamin werden analog Beispiel 1 umgesetzt. Man erhält 5,8 g Öl, die in Diäthyläther gelöst werden und mit 2 g Oxalsäure, die in wenig Äthanol gelöst sind, gefällt. Man erhält 2,7 g (26% d. Th.) farblose Kristalle. Fp. 170–174°C.

$C_{23}H_{30}N_2O_7$ (446,5)

ber.: C 61,9 H 6,8 N 6,3
gef.: C 62,9 H 7,0 N 6,2

Beispiel 12
3-tert.-Butyl-5-[2-(2-Hydroxy-3-tert.-butylamino-propoxy)-styryl]-isoxazol:

7 g (0,023 Mol) 3-tert.-Butyl-5-[2-(2,3-epoxy-propoxy)-styryl]-isoxazol und 3,4 g (0,047 Mol) tert.-Butylamin werden analog Beispiel 4 umgesetzt. Man erhält 6,8 g (72% d. Th.) farblose Kristalle. Fp. 207–208°C.

$C_{22}H_{33}ClN_2O_3$ (408,9)

ber.: C 64,6 H 8,1 Cl 8,7 N 6,9
gef.: C 64,2 H 8,0 Cl 8,6 N 6,7

Beispiel 13
3-Methyl-5-[2-(2-hydroxy-3-(3-methyl-1-butin-3-ylamino)-propoxy)-styryl]-isoxazol:

5 g (0,019 Mol) 3-Methyl-5-[2-(2,3-epoxy-propoxy)-styryl]-isoxazol und 1,7 g (0,02 Mol) 3-Methyl-3-amino-butin-1 werden analog Beispiel 3 umgesetzt und wie dort beschrieben in das Hydrochlorid überführt.

Ausbeute: 3,5 g (49% d. Th.) Fp. 191°C.

$C_{20}H_{25}ClO_3$ (376,9)

| | | | | |
|---|---|---|---|---|
| ber.: | C 63,7 | H 6,7 | N 7,4 | Cl 9,4 |
| gef.: | C 63,7 | H 6,8 | N 7,7 | Cl 9,5 |

Beispiel 14
3-Methyl-5-[2-(2-hydroxy-3-tert.-butylaminopropoxy)-styryl]-isoxazol-hydrochlorid:

In einem Autoklaven werden 2,4 g 3-Methyl-5-[2-(2-hydroxy-3-chlorpropoxy)-styryl]-isoxazol, 10 ml tert.-Butylamin und 50 ml Dioxan 10 Stunden auf 100°C erhitzt. Nach dem Abdestillieren der flüchtigen Anteile im Vakuum wird der hochviskose Rohaustrag in Äther-2-N-Schwefelsäure verteilt, die Wasserphase vorsichtig mit 4-N-Natronlauge alkalisch gestellt und abschliessend mit Äther extrahiert. Nach dem Trocknen der organischen Phase über Natriumsulfat wird vom Lösungsmittel befreit und der verbleibende Rückstand wie in Beispiel 3 beschrieben in 3-Methyl-5-[2-(2-hydroxy-3-tert.-butylaminopropoxy)-styryl]-isoxazol-hydrochlorid vom Schmelzpunkt 216–218°C überführt.

Formulierungsbeispiele, die in üblicher Weise hergestellt werden:

1. Tabletten

| | |
|---|---|
| a) Ein Wirkstoff der Formel I | 5 mg |
| Lactose | 200 mg |
| Methylcellulose | 15 mg |
| Maisstärke | 50 mg |
| Talkum | 11 mg |
| Magnesiumstearat | 4 mg |
| | 285 mg |

| | |
|---|---|
| b) Ein Wirkstoff der Formel I | 20 mg |
| Lactose | 178 mg |
| Avicel | 80 mg |
| Polywachs 600 | 20 mg |
| Magnesiumstearat | 2 mg |
| | 300 mg |

| | |
|---|---|
| c) Verbindung der Formel I | 50 mg |
| Polyvinylpyrrolidon (mittl. M.G. 25000) | 170 mg |
| Polyäthylenglykol (mittl. M.G. 4000) | 14 mg |
| Hydroxypropylmethylcellulose | 40 mg |
| Talkum | 4 mg |
| Magnesiumstearat | 2 mg |
| | 280 mg |

Zu c):

Der Wirkstoff wird mit Polyvinylpyrrolidon in 10%iger wässriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4000), Hydroxypropylmethylcellulose, Talkum und Magnesiumstearat vermischt und zu Tabletten à 280 mg verpresst.

2. Beispiel für Dragees

| | |
|---|---|
| Verbindung der Formel I | 3 mg |
| Lactose | 90 mg |
| Maisstärke | 60 mg |
| Polyvinylpyrrolidon | 6 mg |
| Magnesiumstearat | 1 mg |
| | 160 mg |

Die Mischung der Wirkstoffsubstanz mit Lactose und Maisstärke wird mit einer 8%igen wässrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpresst. Die erhaltenen Drageekerne werden in üblicher Weise mit einer Hülle überzogen, die im wesentlichen aus Zucker und Talkum besteht.

3. Kapselformulierung

| | |
|---|---|
| Verbindung der Formel I | 5,0 mg |
| Magnesiumstearat | 2,0 mg |
| Milchzucker | 19,3 mg |

4. Injektionslösung

| | |
|---|---|
| Verbindung der Formel I | 10 mg |
| Natriumchlorid | 9 mg |
| destilliertes Wasser, q.s. auf 1,0 ml | |

**Patentansprüche**

1. Verbindungen der allgemeinen Formel I,

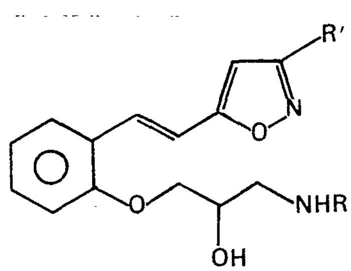

(I)

in der R Isopropyl, tert.-Butyl, einen Cyclopropylrest oder den 3-Methyl-1-butinyl-3-rest und R' einen Alkylrest mit 1 bis 4 C-Atomen bedeuten, und ihre physiologisch verträglichen Säureadditionssalze.

2. 3-Methyl-5-[2-(2-hydroxy-3-isopropylaminopropoxy)-styryl]-isoxazol.

3. 3-Methyl-5-[2-(2-hydroxy-3-tert.-butylamino-propoxy)-styryl]-isoxazol.

4. 3-Äthyl-5-[2-(2-hydroxy-3-tert.-butylaminopropoxy)-styryl]-isoxazol.

5. 3-Äthyl-5-2-(2-hydroxy-3-cyclopropylaminopropoxy)-styryl-isoxazol.

6. 3-Methyl-5-2-(2-hydroxy-3-(3-methyl-1-butin-3-ylamino)-propoxy)-styryl-isoxazol.

7. Verbindung der Formel I nach Ansprüchen 1 bis 6 zur Anwendung als Arzeimittel bei der Hypertonie, der coronaren Herzkrankheit oder von Herzrhythmusstörungen.

8. Verfahren zur Herstellung von Verbindun-

gen der allgemeinen Formel I, nach Ansprüchen 1 bis 6, dadurch gekennzeichnet, dass man ein 3-Alkyl-5-styryl-isoxazol der allgemeinen Formel II,

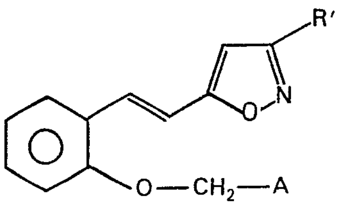

(II)

in der A den Rest

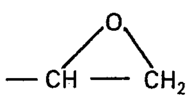

oder

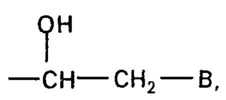

wobei B für eine nukleofuge Abgangsgruppe steht, bedeutet und R′ die für Formel I angegebenen Bedeutungen hat, mit einem Amin der allgemeinen Formel

$H_2N-R$,

in der R die für Formel I angegebenen Bedeutungen hat, zweckmässigerweise in einem Lösungsmittel und gegebenenfalls in Gegenwart eines säurebindenden Mittels bei Temperaturen von 10 bis 120°C umsetzt und gegebenenfalls die erhaltene Verbindung in ein Säureadditionssalz einer physiologisch verträglichen Säure überführt.

9. Therapeutisches Mittel, gekennzeichnet durch einen Gehalt an einer Verbindung der Formel I oder ihrer physiologisch verträglichen Säureadditionssalze nach Ansprüchen 1 bis 6 als Wirkstoff neben üblichen Trägerstoffen und Verdünnungsmitteln.

**Claims**

1. A compound of the general formula I

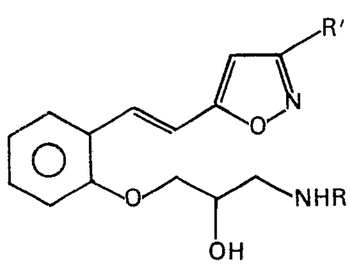

(I)

where R is isopropyl, tert-butyl, cyclopropyl or 3-methyl-but-1-yn-3-yl and R′ is alkyl of 1 to 4 carbon atoms, and its addition salts with physiologically acceptable acids.

2. 3-Methyl-5-[2-(2-hydroxy-3-isopropylamino-propoxy)-styryl]-isoxazole.

3. 3-Methyl-5-[2-(2-hydroxy-3-tert.-butylamino-propoxy)-styryl]-isoxazole.

4. 3-Ethyl-5-[2-(2-hydroxy-3-tert.-butylamino-propoxy)-styryl]-isoxazole.

5. 3-Ethyl-5-[2-(2-hydroxy-3-cyclopropylamino-propoxy)-styryl]-isoxazole.

6. 3-Methyl-5-[2-(2-hydroxy-3-(3-methyl-but-1-ynyl-3-amino-propoxy)-styryl]-isoxazole.

7. A compound of the formula I as claimed in claims 1 to 6 for use as a drug in the case of hypertonia, coronary diseases or cardiac arrhythmias.

8. A process for the preparation of a compound of the general formula I, as claimed in claims 1 to 6, characterized in that a 3-alkyl-5-styryl-isoxazole of the general formula II

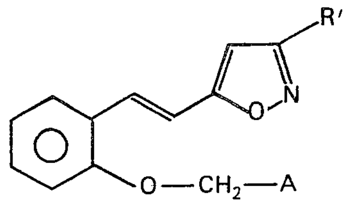

(II)

where A is

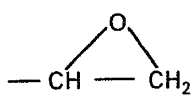

or

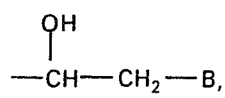

B being a nucleofugic leaving group, and R′ has the meanings given for formula I, is reacted with an amine of the general formula

$'H_2N-R$

where R has the meanings given for formula I, advantageously in a solvent and in the presence or absence of an acid-binding agent, at from 10 to 120°C, and, if required, the resulting compound is converted into an additional salt with a physiologically acceptable acid.

9. A therapeutic agent characterized by a content of a compound of the formula I, or an addition salt thereof with a physiologically acceptable acid as claimed in claims 1 to 6, as the active compound, in addition to conventional excipients and diluents.

**Revendications**

1. Composés de formule générale I

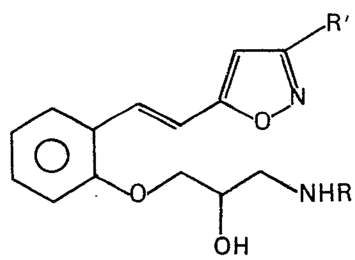

(I)

dans laquelle R représente isopropyle, tert.-butyle, un reste cyclopropyle ou le reste 3-méthyl-1-

butinyle-3 et R′ un reste alkyle à 1 à 4 atomes C, et leurs sels d'addition d'acide compatibles physiologiquement.

2. 3-méthyl-5-[2-(hydroxy-3-isopropylamino-propoxy)-styryl]-isoxazole.

3. 3-méthyl-5-[2-(2-hydroxy-3-tert.-butylamino-propoxy)-styryl]-isoxazole.

4. 3-éthyl-5-[2-(2-hydroxy-3-tert.-butylamino-propoxy)-styryl]-isoxazole.

5. 3-éthyl-5-[2-(2-hydroxy-3-cyclopropylamino-propoxy)-styryl]-isoxazole.

6. 3-méthyl-5-[2-(2-hydroxy-3-(3-méthyl-1-butin-3-yl-amino)-propoxy)styryl]-isoxazole.

7. Composé de formule I selon les revendications 1 à 6 pour utilisation comme médicament dans les cas d'hypertonie, de maladie cardiaque coronarienne et de troubles du système cardiaque.

8. Procédé de préparation de composés de formule générale I, selon les revendications 1 à 6, caractérisé par le fait que l'on fait réagir une 3-alkyl-5-styrylisoxazole de formule générale II

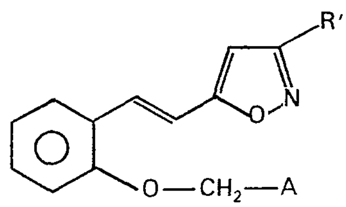

(II)

dans laquelle A représente le reste

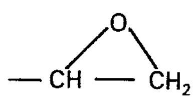

ou

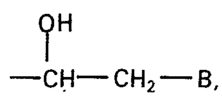

B étant un groupe de départ nucléofuge et R′ a la signification donnée par la formule I, avec une amine de formule générale

$H_2N-R$,

dans laquelle R a la signification donnée par la formule I, de préférence dans un solvent et éventuellement en présence d'un agent liant les acides, à des températures de 10 à 120°C et l'on transforme éventuellement le composé obtenu en un sel d'addition d'acide d'un acide compatible physiologiquement.

9. Agent thérapeutique caractérisé par une teneur en un composé de formule I ou son sel d'addition d'acide compatible physiologiquement, selon les revendications 1 à 6, en tant que principe actif, à côté des véhicules et diluants usuels.